# EUROPEAN PATENT APPLICATION

(11) **EP 4 295 836 A1**
(43) Date of publication of application: **27.12.2023**
(21) Application number: 22386038.8
(22) Date of filing: 22.06.2022
(51) Int. Cl.: A61K 9/00, A61K 31/785, A61K 47/38

(54) **SACHET COMPRISING A LIQUID SUSPENSION OF A SEVELAMER SALT**

(71) Applicant: LABOMED PHARMACEUTICAL COMPANY S.A., 19441 Koropi, Attica (GR)
(72) Inventor: Pachis, Konstantinos, 15343 Agia Paraskevi Attica (GR); Panagiotopoulos, Dimitrios-Tsampikos, 15124 Marousi Attica (GR); Liolios, Georgios, 15232 Chalandri Attica (GR)
(74) Representative: Roukounas, Dimitrios

(57) **Abstract**

A sealed sachet comprising a liquid pharmaceutical suspension composition of a pharmaceutically acceptable salt of sevelamer suitable for oral administration, wherein the inner layer of the sachet is made of a plastic material consisting essentially of a synthetic organic polymer.

## Description

### TECHNICAL FIELD

The present invention relates to a package comprising a liquid pharmaceutical suspension suitable for oral administration comprising a pharmaceutically acceptable salt of sevelamer.

### BACKGROUND OF THE INVENTION

Sevelamer is a non-absorbed phosphate binding polymer used for the control of serum phosphorus in patients with chronic kidney disease (CKD). It comprises polyallyl amine which is crosslinked with epichlorohydrine and its chemical structure is presented in Formula I.

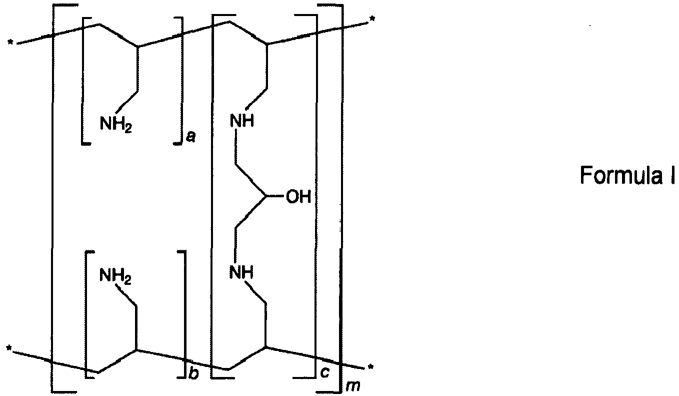

In Formula I above, the sum of a and b, which are the primary amine groups, is 9; c, which designates the number of crosslinking groups, is 1 and m is a large number, which indicates an extended polymer network.

Sevelamer contains multiple amines that become partially protonated in the intestine and interact with phosphate ions through ionic and hydrogen bonding. By binding phosphate in the gastrointestinal tract and thus facilitating phosphorus excretion in feces, sevelamer lowers the plasma phosphorus concentration. Sevelamer, its pharmaceutical compositions, and processes for its preparation were disclosed in US5496545, US5667775, US6509013, US7014846 and US7459151.

Sevelamer may form acid addition salts and is currently marketed either as sevelamer carbonate (Renvela^{®}) and/or as sevelamer hydrochloride (Renagel^{®}).

Patent application WO2007/035313 discloses a powder composition of sevelamer carbonate that can be mixed with water and administered orally as a drink (solution or suspension) while providing acceptable mouth-feel and taste. According to WO2007/035313, the taste problem is solved by mixing sevelamer carbonate with an anionic polymer stabilizer, such as a C₂-C₅ diol ester of alginate, preferably propylene glycol alginate (PGA).

Patent application WO2012/107083 discloses that batches of sevelamer tablets, when packed in a plastic container and tested for long-term stability, particularly at an elevated temperature and environmental moisture content, often exhibit an unpleasant smell at opening the container after a certain time. According to WO2012/107083, the smell problem is solved by using silica gel within a package comprising a sevelamer containing pharmaceutical composition.

Patent application WO2013/064193 discloses that a "chemical smell" of sevelamer carbonate containing powder compositions may appear during opening and emptying of some sachet containers. According to WO2013/064193, the proper selection of the particle size distribution of sevelamer carbonate solves the smell problem.

Patent application KR10-1853260 discloses sevelamer carbonate containing ready-to-use liquid suspension compositions for oral administration and a preparation method thereof. The compositions comprise sevelamer carbonate as an active ingredient, and contain a stabilizer such as a mixture of microcrystalline cellulose/carboxy methylcellulose sodium, and other excipients such as methylcellulose, xanthan gum, and concentrated glycerin.

Patent application WO2020/138921 also discloses ready-to-use sevelamer containing liquid suspension compositions for oral administration and a preparation method therefor. The compositions comprise a suspending agent, a stabilizing agent, a solubilizing adjuvant, and a plasticizer.

Although the problem of improving organoleptic properties of sevelamer formulated in solid state (e.g. tablets, powder for oral suspensions) has been addressed in the prior art, the problem of improving organoleptic properties in sevelamer containing liquid suspension compositions for oral administration has not been addressed.

This is the main reason that currently there are no marketed ready-to-use liquid suspension compositions for oral administration comprising a pharmaceutically acceptable salt of sevelamer.

Thus, there exists a need of a certain improvement in the art. In particular, there exists an objective need of improvement of organoleptic properties, particularly in terms of smell, of liquid suspension compositions for oral administration comprising a pharmaceutically acceptable salt of sevelamer.

### SUMMARY OF THE INVENTION

The present invention provides a sealed sachet comprising a liquid suspension composition for oral administration comprising a pharmaceutically acceptable salt of sevelamer.

With the present invention the organoleptic properties of a liquid suspension composition comprising a pharmaceutical acceptable salt of sevelamer are better than when the same liquid suspension is packaged in alternative containers, such as paper bags; powder bags of plastic films or metal foils; bottles, such as glass, plastic or metal bottles; tubs; or ampules.

With the present invention, such improvement of a liquid suspension comprising a pharmaceutical acceptable salt of sevelamer, does not affect the activity and safety of the active ingredient, but only prevents potential patient's discomfort when using the liquid suspension, particularly when using the liquid suspension that is close to its expiration time.

### DETAILED DESCRIPTION OF THE INVENTION

The present invention provides a sealed sachet comprising a pharmaceutical composition in the form of liquid suspension for oral administration comprising a pharmaceutically acceptable salt of sevelamer, wherein the inner layer of the sachet is made of a plastic material consisting essentially of a synthetic organic polymer.

The term "sealed sachet" as used herein, refers to a sachet that is hermetically sealed by heat or other means. The term "heat-sealed" sachet as used herein refers to a sachet, where the two sheets which form the opening of the sachet are sealed along their edges using heat. Heat sealing offers advanced protection against moisture and oxygen.

The term "inner layer" as used herein in relation to the sachet, refers to the layer of the sachet which is in contact with the suspension composition.

The term "outer layer" as used herein in relation to the sachet, refers to the layer of the sachet which is in immediate contact with the outside environment (atmosphere).

The term "intermediate layer" as used herein in relation to the sachet, refers to a layer of the sachet which is neither inner layer nor outer layer.

The term "ready-to-use" oral liquid suspension as used herein, refers to a liquid suspension ready to be orally administered to a patient.

The common packaging presentations for commercial oral liquid suspension products are multidose containers, such as glass bottles, plastic bottles (e.g. polypropylene bottles, low density polyethylene bottles), tubs or ampules.

The inventors' early research confirmed that a pharmaceutically acceptable salt of sevelamer, when formulated as liquid and filled in such multidose containers, exhibits an unpleasant smell at opening the container after a certain time.

Numerous combinations of excipients commonly used in preparing liquid suspension compositions, such as wetting agents, suspending agents, stabilizing agents, antimicrobial preservatives, antioxidants, viscosity adjusting agents, flavouring agents and sweeteners, were evaluated but did not provide an improvement regarding the unpleasant smell of sevelamer.

After intensive testing it has surprisingly been found that when a liquid suspension composition comprising a pharmaceutically acceptable salt of sevelamer is packaged/stored in a sealed sachet, wherein the inner layer of the sachet, i.e., the layer which is in contact with the suspension composition, is made of a plastic material consisting essentially of a synthetic organic polymer, it is possible to manufacture a liquid suspension having advanced organoleptic properties, particularly in terms of smell.

The synthetic organic polymer according to the present invention is preferably selected from the group consisting of Polyethylene terephthalate (PET), High Density Polyethylene (HDPE), Low Density Polyethylene (LDPE), Linear Low Density Polyethylene (LLDPE), Polypropylene (PP), Polypropylene copolymer (PPCO), Polycarbonate (PC), Polyvinyl chloride (PVC), Polystyrene (PS), Phenol-formaldehyde (PF) and Fluoropolymer (FEP). More preferably, the synthetic organic polymer according to the present invention is selected from the group consisting of PP, HDPE, LLDPE and LDPE. Most preferably, the synthetic organic polymer according to the present invention is polypropylene or LDPE.

The sealed sachet of the present invention can be a mono-layer sachet or multilayer sachet, i.e., a sachet having multiple layers of different materials. Preferably, the sealed sachet is a multilayer sachet.

Examples of mono-layer sachets according to the present invention include sachets wherein the inner layer of the sachet, which is in contact with the suspension composition, is made of a plastic material consisting essentially of PP (e.g. cast polypropylene, high density biaxially-oriented polypropylene, vacuum metalized cast polypropylene), HDPE, LLDPE, or LDPE.

Examples of two-layers sachets according to the present invention include sachets wherein the inner layer of the sachet is made of a plastic material consisting essentially of PP (e.g. cast polypropylene, high density biaxially-oriented polypropylene, vacuum metalized cast polypropylene), HDPE, LLDPE, or LDPE. Such sachets can have, for example, the structure (from the outer layer to the inner layer) biaxially-oriented polypropylene layer / polyethylene layer, or cast polypropylene layer / polyethylene layer, or polyethylene terephthalate layer / polyethylene layer, or biaxially-oriented polypropylene layer / vacuum metalized cast polypropylene layer.

Examples of three-layer sachets according to the present invention include sachets wherein the inner layer of the sachet is made of a plastic material consisting essentially of PP (e.g. cast polypropylene, high density biaxially-oriented polypropylene, vacuum metalized cast polypropylene), HDPE, LLDPE or LDPE. Such sachets can have, for example the structure (from the outer layer to the inner layer) polyethylene terephthalate layer / aluminium foil / polyethylene layer, or polyethylene terephthalate layer / vacuum metalized polyethylene terephthalate layer / polyethylene layer, or biaxially-oriented polypropylene layer / aluminium foil / polyethylene layer, or polyethylene terephthalate layer / aluminium foil / cast polypropylene layer, or biaxially-oriented polypropylene layer / polyethylene terephthalate layer / polyethylene layer, or polyester layer / aluminium foil / low density polyethylene layer.

Examples of four-layer sachets according to the present invention include sachets wherein the inner layer of the sachet is made of a plastic material consisting essentially of PP (e.g. cast polypropylene, high density biaxially-oriented polypropylene, vacuum metalized cast polypropylene), HDPE, LLDPE, or LDPE. Such sachets can have, for example, the structure (from the outer layer to the inner layer) paper layer / low density polyethylene layer / aluminium foil / low density polyethylene layer, or aluminium foil / ethylene methacrylic acid copolymer layer / polyester layer / low density polyethylene layer.

Preferably, the sachet according to the invention has a Water Vapour Transmission Rate which is less than 1 g/m²/day. More preferably the sachet has a Water Vapour Transmission Rate which is less than 0.1 g/m²/day. Even more preferably, the sachet has a Water Vapour Transmission Rate which is less than 0.01 g/m²/day.

The term "Water Vapour Transmission Rate (WVTR)" or "Moisture Vapour Transmission Rate (MVTR)" as used herein, refers to the rate at which water vapour will permeate through solid material over a specific period of time and is measured according to DIN ISO 15106-3 standard at 38°C / 90% Relative Humidity (RH).

Preferably, the sachet according to the invention has an Oxygen Transmission Rate (OTR), which is less than 0.5 cm³/m²/day. More preferably the sachet has an Oxygen Water Vapour Transmission Rate which is less than 0.05 cm³/m²/day. Even more preferably, the sachet has an Oxygen Transmission Rate which is less than 0.005 cm³/m²/day.

The term "Oxygen Transmission Rate (OTR)" is the measurement of the amount of oxygen gas that passes through a substance over a given time period. OTR for plastic film material is the steady state rate at which the oxygen gas permeates through a film at specified conditions (temperature and relative humidity) and is measured according to ASTM D3985 standard at 23°C / 50%RH.

According to a preferred embodiment, the present invention provides a sealed sachet comprising a liquid pharmaceutical suspension composition of a pharmaceutically acceptable salt of sevelamer, wherein the inner layer of the sachets made of a plastic material consisting essentially of LDPE and the sachet has a WVTR, which is less than 0.01 g/m²/day and an OTR, which is less than 0.005 cm³/m²/day.

According to another preferred embodiment, the present invention provides a sealed sachet comprising a liquid pharmaceutical suspension composition of a pharmaceutically acceptable salt of sevelamer, wherein the sachet comprises a polyester layer, an aluminium foil and a low density polyethylene layer, the LDPE layer being the inner layer of the sachet, and wherein the sachet has a WVTR, which is less than 0.01 g/m²/day and an OTR, which is less than 0.005 cm³/m²/day.

The oral pharmaceutical suspension according to the invention may comprise any pharmaceutically acceptable salt of sevelamer. Preferably, the pharmaceutically acceptable salt according to the invention is carbonate, or oxalate, or di-p-toluoyl-L-tartrate, or hydrochloride. More preferably, the pharmaceutically acceptable salt according to the invention is carbonate.

Preferably, the oral pharmaceutical suspension according to the invention comprises from 400 mg/ml to 1400 mg/ml of a pharmaceutically acceptable salt of sevelamer. More preferably, the suspension comprises from 500 mg/ml to 1200 mg/ml of a pharmaceutically acceptable salt of sevelamer. Even more preferably, the suspension comprises from 600 mg/ml to 1000 mg/ml of a pharmaceutically acceptable salt of sevelamer.

Preferably, the oral pharmaceutical suspension according to the invention comprises from 400 mg/ml to 1400 mg/ml of sevelamer carbonate. More preferably, the suspension comprises from 500 mg/ml to 1200 mg/ml of sevelamer carbonate. Even more preferably, the suspension comprises from 600 mg/ml to 1000 mg/ml of sevelamer carbonate.

The oral pharmaceutical suspension according to the invention may optionally contain additional excipients commonly used in preparing liquid suspension compositions, such as a wetting agent, a suspending agent, a stabilizing agent, an antifoaming agent, an antimicrobial preservative, an antioxidant, a viscosity adjusting agent, a flavouring agent or a sweetener.

Examples of wetting agents as referred to herein include but are not limited to sodium lauryl sulfate, docusate sodium and polysorbate 80.

Examples of suspending agents as referred to herein include but are not limited to methylcellulose, hydroxypropyl methylcellulose, sodium carboxymethylcellulose microcrystalline cellulose with sodium carboxymethylcellulose, magnesium aluminium silicate, carbomer, povidone, xanthan gum, carrageenan and polysorbate.

Examples of stabilizing agents as referred to herein include but are not limited to calcium hydroxide and potassium hydroxide.

Example of antifoaming agent as referred to herein include but are not limited to simethicone.

Examples of antimicrobial preservatives as referred to herein include but are not limited to sodium benzoate, benzoic acid, boric acid, sorbic acid and salts thereof, benzyl alcohol, parahydroxy benzoic acids and their alkyl esters, methyl, ethyl and propyl parahydroxy benzoates or their salts, or a mixture thereof.

Examples of antioxidants as referred to herein include but are not limited to sodium metabisulfite, butylated hydroxyanisole, butylated hydroxytoluene, ethylenediamine tetraacetic acid, ascorbic acid, α-tocopherol, propyl gallate, or a mixture thereof.

Examples of viscosity adjusting agents as referred to herein include but are not limited to povidone, sodium carboxymethylcellulose, polyvinylpyrrolidone compounds or a mixture thereof.

Examples of flavouring agents as referred to herein include but are not limited to fruit flavours such as orange, banana, strawberry, cherry, wild cherry, lemon and the like and other flavourings, such as cardamom, anise, mint, menthol, vanillin, bubble gum, or a mixture thereof.

Examples of sweeteners as referred to herein include but are not limited to sugars, i.e. carbohydrates, such as sucrose, glucose, dextrose, lactose, fructose and galactose as well as other sweetening agents known in the art such as sucralose, aspartame, acesulfame-K, thaumatin, mogroside, saccharin and salts thereof, sodium cyclamate, erythritol, glycyrrhizin, monosodium glycyrrhizinate, monoamonium glycyrrhizinate, or a mixture thereof.

The oral suspension of a pharmaceutically acceptable salt of sevelamer, according to the present invention may be supplied as monodose or multidose preparation. Preferably the oral suspension according to the invention is supplied as a monodose preparation.

According to a preferred embodiment, the suspension is a ready-to-use suspension.

The sealed sachet according to the present invention has preferably a volume of less than 50 ml, more preferably less than 40 ml and even more preferably less than 30 ml.

The oral pharmaceutical suspension of the present invention may be prepared using methods well known in the art and using regular manufacturing equipment.

For example, it may be prepared using the following process:
Purified water is added to a vessel under mild stirring. All excipients (e.g. wetting agent, stabilizing agent, antimicrobial preservative, antioxidant, viscosity adjusting agent, flavouring agent or sweetener) except for the suspending agent are successively added to the main vessel under continuous stirring until dissolution of all excipients is achieved.

The suspending agent is then added under vigorous stirring until complete dispersion. The sevelamer salt is added under continuous stirring until a homogeneous dispersion is achieved. Finally, and under vigorous stirring purified water is added to fix the final volume. The final liquid suspension is then filled into sachets, for example, by using a sachet liquid filling machine.

The sealing of the sachets may be performed using processes well known in the art, such as the following process;

The top of the filled sachet is placed between the seal bars of a heat-sealing machine. The sealed bars are pressed down for approximately 2-3 seconds for heat sealing to be applied. The heat-sealed sachet is then released from the heat-sealing machine.

The present invention also provides a pharmaceutical package including a sealed sachet as disclosed above and a leaflet containing information and instructions to a patient for the use of the sevelamer oral suspension.

### EXAMPLES

The following examples show the influence of the packaging on the smell of a suspension comprising sevelamer carbonate.

### EXAMPLE 1

Six liquid suspension compositions disclosed in WO2020/138921 (1, 2, 4, 16, 17 & 22) were prepared through a manufacturing process, where, purified water (100 mL) was heated to 80±5°C and added to a homogenizer. Then propylene glycol (if present), glycerol (if present) and polyoxyl 40 cured castor oil (if present) were added to the homogenizer under continuous stirring (Solution A). Xanthan gum and/or Alginic acid were then added to solution A under continuous stirring. Microcrystalline cellulose (if present), carbomer (if present), methyl paraben and propyl paraben were then added under continuous stirring. Simethicone and sucralose (if present) were added under continuous stirring (solution B). After cooling solution B to 50±5°C, sevelamer carbonate

(8 g as anhydride) was added under continuous stirring until fully dispersed (solution C). After cooling solution C to 35 ± 5°C the flavoring agent was added. Finally, a quantity of purified water up to 200 mL was added.

These compositions (10 ml of the suspension prepared) were packaged in five different packaging systems;
1. Amber type III glass 50 ml bottles sealed with child resistant, tamper evident screw caps
2. Polypropylene 50 ml bottles sealed with child resistant, tamper evident screw caps
3. Heat-sealed 25 ml sachet, comprising the following layers (from outer layer to inner layer): 12 mm Polyester (PES) layer / adhesive layer / 6.35 mm aluminium (ALU) foil / adhesive layer / 80 mm low density polyethylene (LDPE) layer. The OTR of the sachet, measured according to ASTM D3985, is less than 0.005 cm³/m²/day and the WVTR, measured according to DIN ISO 15106-3, is less than 0.01 g/m²/day.
4. The previous sachet (25 ml), which was however unsealed and wrapped with aluminium foil.
5. Heat-sealed aluminium monolayer 25 ml sachet.

**Table 1**

| Ingredient | Function | Composition described in WO 2020/138921 mg/ml | | | | | |
|---|---|---|---|---|---|---|---|
| | | **1** | **2** | **4** | **16** | **17** | **22** |
| Sevelamer carbonate | API | 8000 | 8000 | 8000 | 8000 | 8000 | 8000 |
| Methyl paraben | Preservative | 10 | 10 | 10 | 10 | 10 | 10 |
| Propyl paraben | Preservative | 10 | 10 | 10 | 10 | 10 | 10 |
| Xanthan gum | Suspending agent | 600 | 800 | 800 | 800 | 300 | - |
| Alginic acid | Suspending agent | - | - | - | - | 4000 | 1000 |
| Microcrystalline cellulose | Suspending agent | 3000 | 3000 | 2000 | 3000 | - | 2000 |
| Carbo mer | Suspending agent | 100 | 100 | 100 | 100 | - | - |
| Polyoxyl 40 hardened castor oil | Solubilizer | 3000 | 3000 | 3000 | 3000 | - | - |
| Propylene glycol | Stabilizing agent | 40000 | 40000 | 40000 | 60000 | - | - |
| Glycerol | Stabilizing agent | - | - | - | - | 4000 | 4000 |
| Simethicone | Antifoaming agent | 250 | 250 | 250 | 250 | 50 | 50 |
| Sucralose | Sweetener | 200 | 200 | 200 | 200 | - | - |
| Peppermint oil | Flavor | 10 | 10 | 10 | 10 | 10 | 10 |
| Purified water | Solvent | Qs to 200 mL | | | | | |

The packages were stored at a temperature of 25°C and at a relative humidity of 60% for a total period of six months.

The compositions were studied in relation to their odour (smell intensity). The odour test results were based on the analysis of five trained judges who are experienced in detailed odour analysis.

The descriptive analysis methodology is shown in the following Table 2. The test results are illustrated in the following Table 3.

**Table 2: Odour (smell intense) scale**

| **Scale** | 0 to 1 | 1.1 to 3 | 3.1 to 6 | 6.1 to 9 | 9.1 to 12.0 | 12.1 to 15 |
|---|---|---|---|---|---|---|
| **Description** | Very slight | Slight | Slight to moderate | Moderate | Moderate to high | High |

**Table 3. Average smell intense score**

| **Trial No** | **Test** | **Glass bottle ^{[1]}** | **Polypropylene bottle ^{[2]}** | **Heat sealed Sachet ^{[3]}** | **Unsealed Sachet, wrapped ^{[4]}** | **Heat sealed ALU Sachet [3]** |
|---|---|---|---|---|---|---|
| 1 | Average smell intense score (T= 0) | 0.6 | 0.9 | 0.7 | 1.4 | 1.0 |
| | Average smell intense score (T= 6 months) | 4.6 | 5.1 | 1.5 | 4.3 | 4.9 |
| 2 | Average smell intense score (T= 0) | 0.8 | 0.5 | 0.9 | 0.9 | 0.8 |
| | Average smell intense score (T= 6 months) | 4.1 | 5.7 | 2.1 | 5.3 | 3.6 |
| 4 | Average smell intense score (T= 0) | 0.7 | 1.1 | 1.2 | 1.1 | - |
| | Average smell intense score (T= 6 months) | 4.3 | 5.2 | 1.7 | 4.4 | - |
| 16 | Average smell intense score (T= 0) | 0.9 | 0.8 | 0.9 | 0.9 | 1.1 |
| | Average smell intense score (T= 6 months) | 4.9 | 5.6 | 1.8 | 3.6 | 4.0 |
| 17 | Average smell intense score (T= 0) | 0.6 | 0.7 | 0.6 | 1.2 | 0.8 |
| | Average smell intense score (T= 6 months) | 6.3 | 5.9 | 2.1 | 3.7 | 4.5 |
| 22 | Average smell intense score (T= 0) | 0.9 | 0.9 | 0.8 | 0.6 | - |
| | Average smell intense score (T= 6 months) | 5.5 | 4.9 | 1.5 | 4.3 | - |

| | | | | | | |
|---|---|---|---|---|---|---|
| ^{[1]} Amber type III glass bottle sealed with child resistant, tamper evident screw cap ^{[2]} Polypropylene bottle sealed with child resistant, tamper evident screw cap ^{[3]} Heat-sealed PES/ALU/LDPE sachet ^{[4]} Unsealed PES/ALU/LDPE sachet, wrapped with aluminium foil ^{[5]} Heat-sealed aluminium monolayer sachet | | | | | | |

Interestingly all tested sevelamer suspension compositions (1, 2, 4, 16, 17 & 22), when stored in a package other than according to the invention, exhibited an unpleasant smell when opening the container.

On the other hand, when the tested suspension compositions comprising sevelamer carbonate were packaged/stored in a sealed sachet according to the invention, they showed improved properties in terms of smell even after long term storage.

### EXAMPLE 2

A liquid suspension composition disclosed in KR101853260was prepared through a manufacturing process, where, purified water, glycerin, methyl parahydroxybenzoate and propyl parahydroxybenzoate are added to a vessel, and the mixture was heated to 60 - 65°C under continuous stirring until complete dissolution. Then, xanthan gum was added under continuous stirring and stirred at 60 to 65°C. Microcrystalline cellulose-carboxymethylcellulose sodium and methylcellulose were added under continuous stirring for 2 hours at the same temperature. Sevelamer carbonate was added under continuous stirring, and completely dispersed using a colloid mill. After cooling to 0°C, citric acid hydrate and sodium citrate hydrate were added to an amount of purified water. The resulting solution was slowly added until the pH of the dispersion reached 5.0 and the dispersion was further stirred for 15 minutes. The dispersion was cooled slowly to 35°C and purified water was added up to final volume of 3000 ml. Finally, the liquid suspension was stirred for another 30 minutes.

**Table 4**

| Composition as described in KR101853260 | |
|---|---|
| Ingredient | Amount |
| Sevelamer carbonate | 80.0g |
| Xanthan gum | 0.80g |
| Microcrystalline cellulose, carboxymethylcellulose sodium | 1.10g |
| Methyl cellulose | 0.84g |
| Citric acid hydrate | 0.06g |
| Sodium citrate hydrate | 0.06g |
| Methyl paraoxybenzoate | 25.0mg |
| Propyl paraoxybenzoate | 6.4mg |
| Concentrated glycerin | 5.0g |
| Purified water (q.s. to) | 3000ml |

This composition was packaged in five different packaging systems;
1. Amber type III glass 50 ml bottles sealed with child resistant, tamper evident screw caps
2. Polypropylene 50 ml bottles sealed with child resistant, tamper evident screw caps
3. Heat-sealed 25 ml sachet, comprising the following layers (from outer layer to inner layer): 12 mm Polyester (PES) layer / adhesive layer / 6.35 mm aluminium (ALU) foil / adhesive layer / 80 mm low density polyethylene (LDPE) layer. The OTR of the sachet, measured according to ASTM D3985, is less than 0.005 cm/m²/day and the WVTR, measured according to DIN ISO 15106-3 is less than 0.01 g/m²/day.
4. The previous sachet (25 ml), which was however unsealed and wrapped with aluminium foil.
5. Heat-sealed 25 ml sachet, comprising the following layers (from outer layer to inner layer): 60 mm cast polypropylene (CPP) layer / adhesive layer / 6.35 mm aluminium (ALU) foil / adhesive layer / 6 mm printed polyethylene terephthalate (PET) layer. The OTR of the sachet, measured according to ASTM D3985 is less than 0.01 cm³/m²/day and the WVTR, measured according to DIN ISO 15106-3 is less than 0:02 g/m²/day.

The packages were stored at a temperature of 25°C and at a relative humidity of 60% for a total period of six months.

The composition was studied in relation to its odour (smell intensity). The odour test results were based on the analysis of five trained judges who are experienced in detailed odour analysis.

The descriptive analysis methodology is shown in the following Table 5. The test results are illustrated in the following Table 6.

**Table 5: Odour (smell intense) scale**

| **Scale** | 0 to 1 | 1.1 to 3 | 3.1 to 6 | 6.1 to 9 | 9.1 to 12.0 | 12.1 to 15 |
|---|---|---|---|---|---|---|
| **Description** | Very slight | Slight | Slight to moderate | Moderate | Moderate to high | High |

**Table 6. Average smell intense score**

| **Trial No** | **Test** | **Glass bottle ^{[1]}** | **Polypropylene bottle ^{[2]}** | **Heat sealed Sachet ^{[3]}** | **Unsealed Sachet, wrapped ^{[4]}** | **Heat sealed Sachet ^{[5]}** |
|---|---|---|---|---|---|---|
| 1 | Average smell intense score (T= 0) | 0.7 | 0.6 | 0.9 | 1.0 | 0.8 |
| | Average smell intense score (T= 6m) | 5.6 | 5.0 | 1.2 | 6.3 | 1.9 |

| | | | | | | |
|---|---|---|---|---|---|---|
| ^{[1]} Amber type III glass 50 ml bottles sealed with child resistant, tamper evident screw caps ^{[2]} Polypropylene 50 ml bottles sealed with child resistant, tamper evident screw caps ^{[3]} Heat-sealed 25 ml sachet (PES/ALU/LDPE as per Figure 4) ^{[4]} Unsealed 25 ml sachet, wrapped with aluminium foil ^{[5]} Heat-sealed sachet (PET/ALU/CPP as per Figure 5). | | | | | | |

The above results clearly show that when the composition is stored in a sachet according to the present invention the intensity of the smell is far lower compared to the intensity of the smell when the composition is stored in an alternative container.

### EXAMPLE 3

A liquid suspension composition was prepared through a manufacturing process, where, purified water was added to a vessel under mild stirring. Benzalkonium chloride, sodium chloride, sucralose and peppermint flavour were successively added to the main vessel under continuous stirring until dissolution of all excipients was achieved. The suspending agent (Vivapur^{®} MCG 591P) was then added under vigorous stirring until complete dispersion. Sevelamer carbonate was added under continuous stirring until a homogeneous dispersion was obtained. Finally, purified water was added under vigorous stirring to fix the final volume.

**Table 7**

| Ingredient | Amount |
|---|---|
| Sevelamer carbonate | 24.0 g |
| Vivapur^{®} MCG 591 P (Microcrystalline cellulose, carboxymethylcellulose sodium) | 20 mg/mL |
| Benzalkonium Chloride | 0.1 mg/mL |
| NaCl | 1.5 mg/mL |
| Sucralose | 0.5 mg/mL |
| Peppermint Flavour | 1.0 mg/mL |
| Purified water (q.s. to) | 300 mL |

10 ml of this liquid suspension) were packaged in the five different packaging systems, as described in Example 2.

The packages were store at a temperature of 25°C and a relative humidity of 60% for a total period of six months.

The composition was studied in relation to its odour (smell intensity). The odour test results were based on the analysis of five trained judges who are experienced in detailed odour analysis. The descriptive analysis methodology is shown in the following Table 8. The test results are illustrated in the following Table 9.

**Table 8: Odour (smell intense) scale**

| **Scale** | 0 to 1 | 1.1 to 3 | 3.1 to 6 | 6.1 to 9 | 9.1 to 12.0 | 12.1 to 15 |
|---|---|---|---|---|---|---|
| **Description** | Very slight | Slight | Slight to moderate | Moderate | Moderate to high | High |

**Table 9. Average smell intense score**

| **Trial No** | **Test** | **Glass bottle ^{[1]}** | **Polypropylene bottle ^{[2]}** | **Heat sealed Sachet ^{[3]}** | **Unsealed Sachet, wrapped ^{[4]}** | **Heat sealed Sachet ^{[5]}** |
|---|---|---|---|---|---|---|
| 1 | Average smell intense score (T= 0) | 0.9 | 1.1 | 0.6 | 1.0 | 0.5 |
| | Average smell intense score (T= 6m) | 5.4 | 5.0 | 1.1 | 6.4 | 1.6 |

| | | | | | | |
|---|---|---|---|---|---|---|
| ^{[1]} Amber type III glass 50 ml bottles sealed with child resistant, tamper evident screw caps ^{[2]} Polypropylene 50 ml bottles sealed with child resistant, tamper evident screw caps ^{[3]} Heat-sealed 25 ml sachet (PES/ALU/LDPE) ^{[4]} Unsealed 25 ml sachet, wrapped with aluminium foil ^{[5]} Heat-sealed sachet (PET/ALU/CPP). | | | | | | |

The above results clearly show that when the composition is stored in a sachet according to the present invention the intensity of the smell is far lower compared to the intensity of the smell when the composition is stored in an alternative container.

## Claims

1. A sealed sachet comprising a liquid pharmaceutical suspension composition of a pharmaceutically acceptable salt of sevelamer suitable for oral administration, wherein the inner layer of the sachet is made of a plastic material consisting essentially of a synthetic organic polymer.

2. The sealed sachet according to claim 1, wherein the synthetic organic polymer is selected form the group consisting of polyethylene terephthalate, high density polyethylene, low density polyethylene, linear low-density polyethylene, polypropylene, polypropylene copolymer, polycarbonate, polyvinyl chloride, polystyrene, phenol-formaldehyde and fluoropolymer.

3. The sealed sachet according to claim 1 or 2, wherein the synthetic organic polymer is selected from the group consisting of polypropylene, high density polyethylene, linear low-density polyethylene and low density polyethylene.

4. The sealed sachet according to any one of the preceding claims, wherein the synthetic organic polymer is cast polypropylene, or low density polyethylene.

5. The sealed sachet according to any one of the preceding claims, wherein the sachet has a water vapour transmission rate of less than 0.1 g/m²/day, measured according to DIN ISO 15106-3 and an oxygen transmission rate of less than 0.05 cm³/m²/day, measured according to ASTM D3985.

6. The sealed sachet according to any one of the preceding claims, wherein the sachet has a water vapour transmission rate of less than 0.01 g/m²/day, measured according to DIN ISO 15106-3 and an oxygen transmission rate of less than 0.005 cm³/m2/day, measured according to ASTM D3985.

7. The sealed sachet according to any one of the preceding claims, wherein the sachet is heat sealed.

8. The sealed sachet according to any one of the preceding claims, wherein the sachet is a multilayer sachet.

9. The sealed sachet according to any one of the preceding claims, wherein the sachet comprises
a polyester layer as outer layer,
an aluminium foil as intermediate layer,
a low density polyethylene layer as inner layer,
wherein the oxygen transmission rate of the sachet is less than 0.005 cm³/m²/day and the water vapour transmission rate of the sachet is less than 0.01 g/m²/day.

10. The sealed sachet according to any one of the preceding claims, wherein the volume of the sachet is less than 30 ml.

11. The sealed sachet according to any one of the preceding claims, wherein the composition is aqueous.

12. The sealed sachet according to any one of the preceding claims, wherein the composition further comprises one or more excipients selected from the group consisting of a wetting agent, a suspending agent, a stabilizing agent, an antifoaming agent, an antimicrobial preservative, an antioxidant, a viscosity adjusting agent, a flavouring agent and a sweetener.

13. The sealed sachet according to any one of the preceding claims, wherein the composition is a ready-to-use suspension.

14. The sealed sachet according to any one of the preceding claims, wherein the composition comprises from 400 mg/ml to 1400 mg/ml of a pharmaceutically acceptable salt of sevelamer.

15. The sealed sachet according to any one of the preceding claims, wherein the pharmaceutically acceptable salt of sevelamer is selected from the group consisting of carbonate, oxalate, di-p-toluoyl-L-tartrate and hydrochloride.
